# EUROPEAN PATENT SPECIFICATION

(11) **EP 1 153 129 B1**
(45) Date of publication and mention of the grant of the patent: **14.10.2009**
(21) Application number: 00905838.9
(22) Date of filing: 29.01.2000
(51) Int. Cl.: C12N 15/11, C07K 14/00, A61K 47/48

(54) **METHOD OF TREATING RESTENOSIS BY ANTISENSE TARGETING OF C-MYC**
VERFAHREN ZUR BEHANDLUNG VON RESTENOSE DURCH AUF C-MYC ZIELGERICHTETE ANTISENSEVERBINDUNGEN
TRAITEMENT DE LA RESTENOSE PAR CIBLAGE ANTISENS DU C-MYC

(30) Priority: 29.01.1999 US 117846 P
(43) Date of publication of application: 14.11.2001
(73) Proprietor: AVI BioPharma, Inc., Corvallis, OR 97333 (US)
(72) Inventor: IVERSEN, Patrick, L., Corvallis, OR 97330 (US); WELLER, Dwight, D., Corvallis, OR 97330 (US)
(74) Representative: Vossius & Partner
(86) International application number: PCT/US2000/002331
(87) International publication number: WO 2000/044897

(56) References cited:
- WO-A-86/05518
- WO-A-91/09033
- WO-A-91/09073
- WO-A-94/15646
- WO-A-95/10305
- BENNETT M R ET AL: "INHIBITION OF VASCULAR SMOOTH MUSCLE CELL PROLIFERATION IN VITRO AND IN VIVO BY C-MYC ANTISENSE OLIGODEOXYNUCLEOTIDES" JOURNAL OF CLINICAL INVESTIGATION,US,NEW YORK, NY, vol. 93, no. 2, 1 February 1994 (1994-02-01), pages 820-828, XP000574003 ISSN: 0021-9738
- SHI Y ET AL: "TRANSCATHETER DELIVERY OF C-MYC ANTISENSE OLIGOMERS REDUCES NEOINTIMAL FORMATION IN A PORCINE MODEL OF CORONARY ARTERY BALLOON INJURY" CIRCULATION,US,AMERICAN HEART ASSOCIATION, DALLAS, TX, vol. 90, no. 2, 1 August 1994 (1994-08-01), pages 944-951, XP000605220 ISSN: 0009-7322
- BURGESS T L ET AL: "THE ANTIPROLIFERATIVE ACTIVITY OF C-MYB AND C-MYC ANTISENSE OLIGONUCLEOTIDES IN SMOOTH MUSCLE CELLS IS CAUSED BY A NONANTISENSE MECHANISM" PROCEEDINGS OF THE NATIONAL ACADEMY OF SCIENCES OF USA,US,NATIONAL ACADEMY OF SCIENCE. WASHINGTON, vol. 92, no. 9, 25 April 1995 (1995-04-25), pages 4051-4055, XP002015216 ISSN: 0027-8424 cited in the application
- SADATOSHI BIRO ET AL: "INHIBITORY EFFECTS OF ANTISENSE OLIGODEOXYNUCLEOTIDES TARGETING C-MYC MRNA ON SMOOTH MUSCLE CELL PROLIFERATION AND MIGRATION" PROCEEDINGS OF THE NATIONAL ACADEMY OF SCIENCES OF USA,US,NATIONAL ACADEMY OF SCIENCE. WASHINGTON, vol. 90, 1 January 1993 (1993-01-01), pages 654-658, XP002025444 ISSN: 0027-8424

## Description

The present invention relates to morpholino antisense compounds and their use in the preparation of a pharmaceutical composition for inhibiting restenosis in a patient.

### References

Alfke H; et al.; Cardiovasc Intervent Radiol, Jan-Feb 1998, 21 (1) p. 50-6.
Allen RT; et al.; Scanning, Nov 1998, 20 (8) p. 577-86.
Badimon L; et al.; Z Kardiol, 1995; 84 Suppl 4 p. 145-9.
Barath P; et al.; Cathet Cardiovasc Diagn, Jul 1997, 41 (3) p. 333-41.
Bartorelli AL; et al. Cathet Cardiovasc Diag, Nov 1997, 42 (3) p. 313-20.
Bauriedel G; et al., Z Kardiol, 1994, 83 Suppl 4 p. 31-41.
Ben-Yosef, T., et al., Oncogene 17(2):165-71, 1998.
Bennett MR; et al., Biochem J, Sep 15 1994, 302 (Pt 3) p. 701-8.
Bennett MR; et al., J Clin Invest, Feb 1994, 93 (2) p. 820-8.
Burgess TL; et al., Proc Natl Acad Sci USA, Apr 25 1995, 92 (9) p. 4051-5.
Casterella PJ; et al., Cardiol Rev, Jul-Aug 1999, 7 (4) p. 219-31.
Chen SJ; et al., Circulation, Nov 1994, 90 (5) p. 2468-73.
Consigny PM; et al., J Vasc Interv Radiol, Sep-Oct 1994, 5 (5) p. 731-7.
Dev NB; et al., Cathet Cardiovasc Diagn, Nov 1998, 45 (3) p. 337-45.
Dick A; et al., Cardiovasc Intervent Radiol, Sep-Oct 1999, 22 (5) p. 389-93.
Fernandez-Ortiz A; et al., Circulation, Apr. 1994, 89 (4) p.1518-22.
Gottman D; et al., Rofo Fortschr Geb Rontgenstr Neuen Bildgeb Verfahr, Jan 1999, 170 (1) p. 84-8.
Hamon M; et. al., Drugs Aging, Oct 1998, 13 (4) p. 291-301.
Herdeg C; et al., Cathet Cardiovasc Diagn, Jul 1997, 41 (3) p. 308-14.
Hodgkin DD; et al., J Cardiovasc Pharmacol, Jan 1997, 29 (1) p. 39-44.
Hong MK; et al., Cathet Cardiovasc Diagn, Mar 1995, 34 (3) p. 263-70.
Hong MK: et al., Coron Artery Dis, Nov 1993, 4 (11) p. 1023-7.
Imanishi T et al., Jpn. Circ. J., 61(3) 256-262, 1997.
Kimura T; et al., Jpn Circ J, Apr 1998, 62 (4) p. 299-304.
Koh WJ; et al., Int J Radiat Oncol Biol Phys.,, Nov 1 1996, 36 (4) p. 829-34.
Lambert CR; et al., Coron Artery Dis, May 1993, 4 (5) p. 469-75.
Lee M; et al., Antisense Nucleic Acid, Drug Dev Oct 1999, 9 (5); p. 487-92.
Meyer BJ; et al., Circulation, Nov 1994, 90 (5) p2474-80.
Oberhoff M; et al., Cathet Cardiovasc Diagn, Jul 1997, 41 (3) p268-74.
Pavlides GS; et al., Cathet Cardiovasc Diagn, Jul 1997, 41 (3) p287-92.
Porter TR; et al., J Ultrasound Med, Aug 1996, 15(8):577.
Raman VK; et al., Semin Interv Cardiol, Sep-Dec 1998, 3 (3-4) p133-7.
Robinson KA; et al.,Cathet Cardiovasc Diagn, Jul 1997, 41 (3) p354-9.
Robinson KA; et al., Cathet Cardiovasc Diagn, Jul 1997, 41 (3) p348-53.
Roy S; et al., J Vasc Interv Radiol Jun 1999, 10 (6) p817-24.
Rubartelli P; et al., J Am Coll Cardiol, Jul 1998, 32 (1) p90-6.
Savage MP; et al., J Am Coll Cardiol, Feb 1998, 31 (2) p307-11.
Shi Y; et al.; Circulation, Aug. 1994, 90 p. 944-951.
Sirnes PA; et al., Int J Cardiol, Dec 1 1998, 67(2) p. 111-8.
Skaletz-Rorowski A; et al., Arterioscler Thromb Vasc Biol, Jul 1999; 19(7) p. 1608-14.
Teomim D; et al., J Controlled Releas, Jun 28 1999, 60 (1) p129-42.
Wilensky RL; et al., Am Heart J Oct 1991, 122 (4 Pt 1) p1136-40.
WO 94/15646
WO95/10305

Transluminal coronary angioplasty was introduced in the late 1970's as a nonsurgical treatment for obstructive coronary artery disease. Since its introduction, major advances in equipment and techniques have led to widespread use of the method for treating coronary artery disease and angina. Typically, the procedure involves placing a balloon-tip catheter at the site of occlusion, and disrupting and expanding the occluded vessel by inflating the catheter balloon.

Despite improvements in equipment and techniques, restenosis persists as the limiting factor in the maintenance of vessel patency in angioplasty, occurring in 30 % to 50% of patients, and accounting for significant morbidity and health care expenditures. (Casterella). Post-angioplasty restenosis is a segmentally limited, wound healing response to a traumatization of the vascular wall. Studies with animal models and human autopsy plaque tissue indicate a cascade-like course of events triggered by (a) destruction of endothelial and subendothelial structures, (b) traumatization of medial regions with rupture of the internal elastic lamina, (c) release of thrombogenic factors such as collagen or tissue factor, (d) stretching of smooth muscle cells with subsequent expression of proto-oncogenes (c-fos, *c-myc*, c-myb), (e) release of growth factors from cells of the bloodstream, endothelial cells and SMCs, and (f) thrombin production with autocatalytic activation of the SMC thrombin receptor (Bauriedel).

Overlapping the inflammation period, granulation begins 3 days after angioplasty. Proteinases such as plasmin as well as collagenases induce the disintegration of extracellular matrix structures, thereby modulating plaque formation, and lead to an organelle-rich SMC phenotype within the intima and media. Overlapping with the granulation period, induction of different components of the extracellular matrix occurs 1-2 weeks after angioplasty, possibly mediated by TGF-beta (phase of matrix formation). Smooth muscle cells produce and secrete matrix proteins such as tenascin, fibronectin, collagens and proteoglycans, and thereby induce a marked increase of the neointimal plaque volume. (Bauriedel).

Clinical trials in restenosis prevention using various revascularization devices, antiplatelet drugs, antithrombotic drugs, and anti-inflammatory agents have produced limited improvement in the incidence of restenosis. Also reported are attempts to improve the risk or severity of restenosis with intravascular stents (Savage, Eisenhower, Rubarteli, Gottman), radiation therapy (Koh), and administration of antiproliferative drugs at the vessel injury site. The latter approach typically employs the balloon catheter for introducing the therapeutic agent at the vessel occlusion site (Dick, Roy, Dev, Kimura, Alfke, Robinson 1997a, Robinson 1997b, Barath, Herdeg, Pavlides, Oberhoff, Hodgkin, Hong, Consigny, Meyer, Fernadez-Ortiz, Lambert, and Wilensky), or releasing drug from a stent (Teomin, Bartonelli, Raman, Gibson).

Despite these advances, the incidence of restenosis, and the inability to predict the response to treatment, remains a serious risk factor in vascular angioplasty. It would therefore be desirable to (i) provide a treatment method which shows efficacy in reducing the incidence and severity of restenosis following vascular angioplasty, (ii) is well tolerated by the patients, with few or any side effects, and (iii) can be carried out with a variety of therapeutic delivery methods.

Zalewski, Shi et al. (WO 94/15646; WO 95/10305; Circulation 90:944-951 (1994)) describe inhibition of smooth muscle cell proliferation by phosphorothioate-linked oligonucleotides antisense to c-*myc,* in human cells in vitro and in vivo in a porcine model of coronary artery balloon injury. Bennett et al. (J. Clin. Invest. 93(2):820-828 (1994)) describe inhibition of smooth muscle cell proliferation, in rat VSMC cells in vitro and in vivo in a rat model of intimal hyperplasia, using phosphorothioate-linked oligonucleotides antisense to c-*myc*.

It would also be desirable to provide improved therapeutic compounds and compositions for carrying out the method, and a simple, rapid clinical assay for monitoring effectiveness of the delivery of a therapeutic compound to the vessel target site.

### Summary of the Invention

In one aspect, the invention relates to the use of a morpholino antisense compound having:
(i) from 8 to 40 nucleotides,
(ii) morpholino subunits linked together by phosphorodiamidate linkages, 2 atoms long, joining the morpholino nitrogen of one subunit to the 5' exocyclic carbon of an adjacent subunit, and
(iii) a sequence of bases attached to the subunits and containing the sequence identified by SEQ ID NO:1,
for the preparation of a pharmaceutical composition for use in inhibiting restenosis in a patient.

The pharmaceutical composition is to be administered by:
(a) contacting the region of the vessel with a reservoir containing the antisense compound, and introducing the compound from the reservoir into the vessel by iontophoresis or electroporation;
(b) injecting the compound from the catheter directly into the region of the vessel, under pressure, through injectors contained on the surface of the catheter balloon, where said injectors are capable of penetrating the tunica media in the vessel;
(c) injecting into or contacting the region of the vessel, microparticles containing the antisense compound in entrapped form;
(d) contacting the region of the vessel with a hydrogel coating contained on the surface of the catheter balloon, and containing the antisense compound in diffusible form; or
(e) contacting the region of the vessel with a stent having an outer surface layer containing the antisense compound in diffusible form.

The antisense compound preferably has intersubunit linkages of the form: 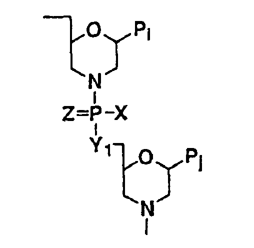 where X is an unsubstituted, monosubstituted or disubstituted nitrogen, Y₁ and Z are oxygen and Pᵢ and Pⱼ are purine or pyrimidine base-pairing moieties effective to bind, by base-specific hydrogen bonding, to a base in a polynucleotide.

The intersubunit linkages are exemplified particularly by the phosphorodiamidate linkage represented at Figure 2B-B, where X=NH₂, Y=O, and Z=O. An exemplary sequence is the one identified by SEQ ID NO:1.

For use in mode of administration (a), the antisense compound is preferably contained in a volume between two inflated balloons in the catheter, and the volume is subjected to pulsed electric fields effective to ionotophoretically drive the compound into region of the vessel.

For use in mode of administration (b), the catheter balloon preferably has a plurality of outer-facing channels that communicate with a distal-tip reservoir, where each channel having one or more injection ports or fingers, and the injecting step includes forcing a solution or suspension of the antisense compound from the reservoir through the injection ports when the balloon is in an inflated position.

For use in mode of administration (c), the catheter preferably has a distal end reservoir, the microparticles are contained as a particle suspension in the reservoir, and the injecting step includes forcing the suspension out of the catheter through a catheter surface in contact with the vessel region. Exemplary particles include biodegradable polymer particles or liposomes with entrapped antisense compounds or microbubbles designed to release entrapped compound when subjected to ultrasonic energy.

For use in mode of mode of administration (d), the hydrogel coating is preferably designed to release the majority of the antisense compound in the coating over a period of 5-60 minutes following balloon angioplasty.

For use in mode (e), the stent may be biodegradable, and designed to release the majority of the antisense compound in the coating over a period of 5-60 minutes following balloon angioplasty.

The antisense compound may be derivatized at its 5' end with triethyleneglycol. The compound is preferably delivered by direct application of the compound to the target vessel region, immediately following balloon angioplasty, or during a coronary bypass operation, from a solution containing at least 30 mg/ml to achieve a final amount of compound administered to the target region of between about 0.5 to 2 mg.

In another aspect, the invention includes a morpholino antisense compound having:
(i) from 8 to 40 nucleotides,
(ii) morpholino subunits linked together by phosphorodiamidate linkages, 2 atoms long, joining the morpholino nitrogen of one subunit to the 5' exocyclic carbon of an adjacent subunit, and
(iii) a sequence of bases attached to the subunits and containing the sequence identified by SEQ ID NO:1.

The antisense compound may be derivatized at its 5' end, with triethyleneglycol. The compound may be included in a liposomal or biodegradable microparticle.

These and other objects and features of the present invention will become more fully apparent when the following detailed description of the invention is read in conjunction with the accompanying drawings.

### Brief Description of the Drawings

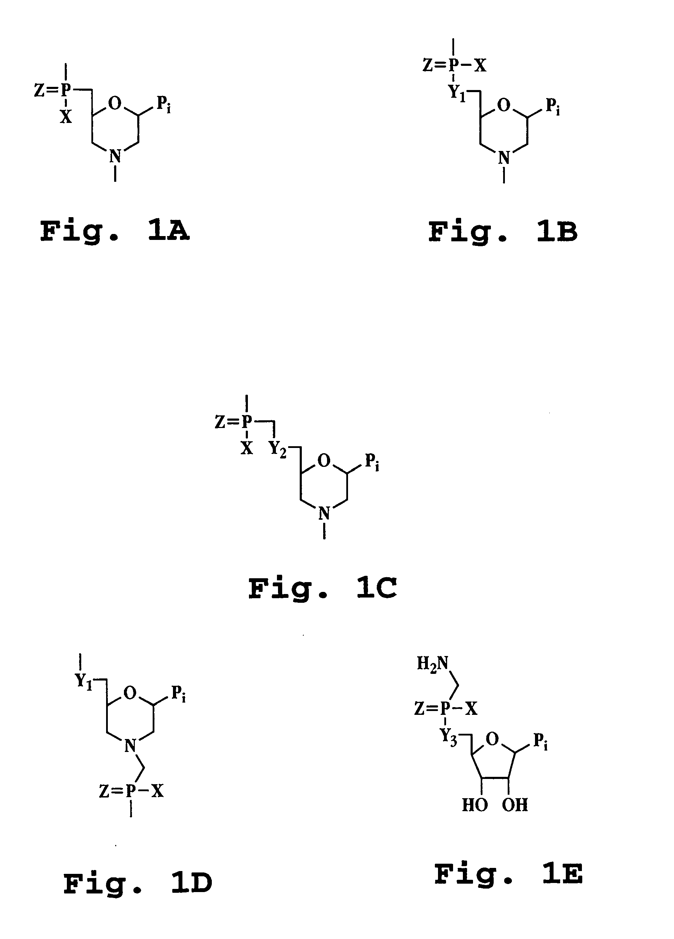

Figure 1 shows several preferred subunits having 5-atom (A), six-atom (B) and seven-atom (C-E) linking groups suitable for forming polymers.
Figures 2A-A to 2E-E show the repeating subunit segment of exemplary morpholino oligonucleotides, designated A-A through E-E, constructed using subunits A-E, respectively, of Figure 1.
Figure 3 is a kinetic representation of the disappearance of PMO monomer and appearance of RNA:PMO heterodimer in the plasma of rats administered the P450 antisense PMO.

### I. Definitions

The terms below, as used herein, have the following meanings, unless indicated otherwise:

"Antisense" refers to an oligomer having a sequence of nucleotide bases and a subunit-to-subunit backbone that allows the antisense oligomer to hybridize to a target sequence in an RNA by Watson-Crick base pairing, to form an RNA:oligomer heteroduplex within the target sequence, typically with an mRNA. The oligomer may have exact sequence complementarity to the target sequence or near complementarity. These antisense oligomers may block or inhibit translation of the mRNA, and/or modify the processing of an mRNA to produce a splice variant of the mRNA. Studies conducted in support of the present invention, for example, show that the antisense compound represented by SEQ ID NO:1 interferes with *c-myc* mRNA processing, leading to a truncated mRNA in which the normal start codon and adjacent region has been spliced out of the mRNA.

As used herein, the terms "compound", "agent", "oligomer" and "oligonucleotide" may be used interchangeably with respect to the antisense oligonucleotides of the invention. As used herein, a "morpholino oligomer" refers to a polymeric molecule having a backbone which supports bases capable of hydrogen bonding to typical polynucleotides, wherein the polymer lacks a pentose sugar backbone moiety, and more specifically a ribose backbone linked by phosphodiester bonds which is typical of nucleotides and nucleosides, but instead contains a ring nitrogen with coupling through the ring nitrogen. A preferred "morpholino" oligonucleotide is composed of morpholino subunit structures of the form shown in Fig. 2B-B, where (i) the structures are linked together by phosphorous-containing linkages, one to three atoms long, joining the morpholino nitrogen of one subunit to the 5' exocyclic carbon of an adjacent subunit, and (ii) Pᵢ and Pⱼ are purine or pyrimidine base-pairing moieties effective to bind, by base-specific hydrogen bonding, to a base in a polynucleotide. Exemplary structures for antisense oligonucleotides for use in the invention include the morpholino subunit types shown in Figures 1A-E, with the linkages shown in Figures 2A-A to 2E-E.

As used herein, a "nuclease-resistant" oligomeric molecule (oligomer) is one whose backbone is not susceptible to nuclease cleavage of a phosphodiester bond.

As used herein, an oligonucleotide or antisense oligomer "specifically hybridizes" to a target polynucleotide if the oligomer hybridizes to the target under physiological conditions, with a Tm substantially greater than 37°C, preferably at least 50° C, and typically 60°C-80°C or higher. At a given ionic strength and pH, the T[m] is the temperature at which 50% of a target sequence hybridizes to a complementary polynucleotide.

Polynucleotides are described as "complementary" to one another when hybridization occurs in an antiparallel configuration between two single-stranded polynucleotides. A double-stranded polynucleotide can be "complementary" to another polynucleotide, if hybridization can occur between one of the strands of the first polynucleotide and the second. Complementarity (the degree that one polynucleotide is complementary with another) is quantifiable in terms of the proportion of bases in opposing strands that are expected to form hydrogen bonds with each other, according to generally accepted base-pairing rules.

As used herein, the term "*c-myc* antisense compound" refers to a nuclease-resistant antisense morpholino compound having high affinity (*i*.*e*., "specifically hybridizes") to a complementary or near-complementary *c-myc* nucleic acid sequence, e. g. , the sequence including and spanning the normal AUG start site.

As used herein the term "analog" in reference to an oligomer means a substance possessing both structural and chemical properties similar to those of the reference oligomer.

As used herein, "effective amount" relative to an antisense oligomer refers to the amount of antisense oligomer to be administered to a mammalian subject, either as a single dose or as part of a series of doses, that is effective to reduce the risk (incidence) or severity (amount of occlusion) of restenosis, following balloon angioplasty.

As used herein, the term "body fluid" encompasses a variety of sample types obtained from a subject including, urine, saliva, plasma, blood, spinal fluid, and other liquid sample of biological origin, and may refer include cells or cell fragments suspended therein, or the liquid medium and its solutes.

### II. Compound and Composition

### A. c-myc Antisense Compound

*c-myc* is a proto-oncogene which regulates cell growth and differentiation, is involved in the process of vascular remodeling, regulating smooth muscle cell proliferation and extracellular matrix synthesis, in addition to playing a role in apoptosis. Aberrant expression of *c-myc* is frequently observed in human cancer. Aberrant, constitutive or overexpression of *c-myc* has been associated with a number of human cancers including lung cancer, colorectal cancer, breast cancer, bladder cancer, leukemia, lung cancer, etc.

Several *in vitro* studies have demonstrated that phosphorothioate oligodeoxynucleotides targeted against genes involved in smooth muscle cell proliferation inhibit both proliferation and migration. In one study *in vivo* administration of phosphorothioate oligonucleotides targeted against *c-myc* using a porous balloon catheter in a porcine coronary artery model (Shi), and in another study phosphorothioate oligonucleotides delivered intraluminally and targeted against c-myb, *c-myc*, cdc2 kinase, cdk2 kinase and proliferating cell nuclear antigen (PCNA) inhibited neointimal formation after balloon injury in both the rat carotid and porcine coronary artery models (Lee).

However, a similar study single endoluminal transcatheter delivery of antisense oligonucleotides directed against cell cycle regulatory proteins using a porous balloon catheter did not affect neointima formation or vessel size (Robinson). The results of a further study using phosphorothioate oligonucleotides directed toward c-myb and *c-myc* indicated inhibition of smooth muscle cell proliferation. However, the observed inhibition was clearly not via an antisense mechanism, but was correlated with the presence of four contiguous guanosine residues in the oligonucleotide sequence *in vitro* in primary cultures of smooth muscle cells and in arteries *ex vivo* (Burgess).

In accordance with the present invention, it has been discovered that a morpholino antisense compound having (i) from 8 to 40 nucleotides, including a targeting base sequence that is complementary to a region that spans the translational start codon of a *c-myc* mRNA and (ii) uncharged, phosphorous-containing intersubunit linkages produces a significant reduction in the incidence and severity of restenosis. *In vitro* and animal-model studies conducted in support of the invention indicate that the antisense compound (i) is taken up efficiently by cells in a vessel lumen which are exposed to the antisense compound, (ii) acts intracellularly to inhibit correct processing (mRNA splicing) and translation of processed *c-myc* mRNA, and (iii) is significantly more effective, in reducing the incidence and severity of restenosis, than other types of *c-myc* antisense compounds, *e*.*g*., phosphorothioate *c-myc* antisense compounds.

The synthesis, structures, and binding characteristics of morpholino oligomers are detailed in above-cited U.S. Patent Nos. 5,698,685, 5,217,866, 5,142,047, 5,034,506, 5,166,315, 5,521,063, and 5,506,337. The antisense oligomers (compounds) of the present invention are composed of morpholino subunits of the form shown in the above cited patents, where (i) the morpholino groups are linked together by uncharged phosphorus-containing linkages, one to three atoms long, joining the morpholino nitrogen of one subunit to the 5' exocyclic carbon of an adjacent subunit, and (ii) the base attached to the morpholino group is a purine or pyrimidine base-pairing moiety effective to bind, by base-specific hydrogen bonding, to a base in a polynucleotide. The purine or pyrimidine base-pairing moiety is typically adenine, cytosine, guanine, uracil or thymine. Preparation of such oligomers is described in detail in U.S. Patent No. 5,185,444 (Summerton and Weller, 1993). As shown in the reference, several types of nonionic linkages may be used to construct a morpholino backbone.

Exemplary backbone structures for antisense oligonucleotides of the invention include the β-morpholino subunit types shown in Fig 1A-E. It will be appreciated that a polynucleotide may contain more than one linkage type.

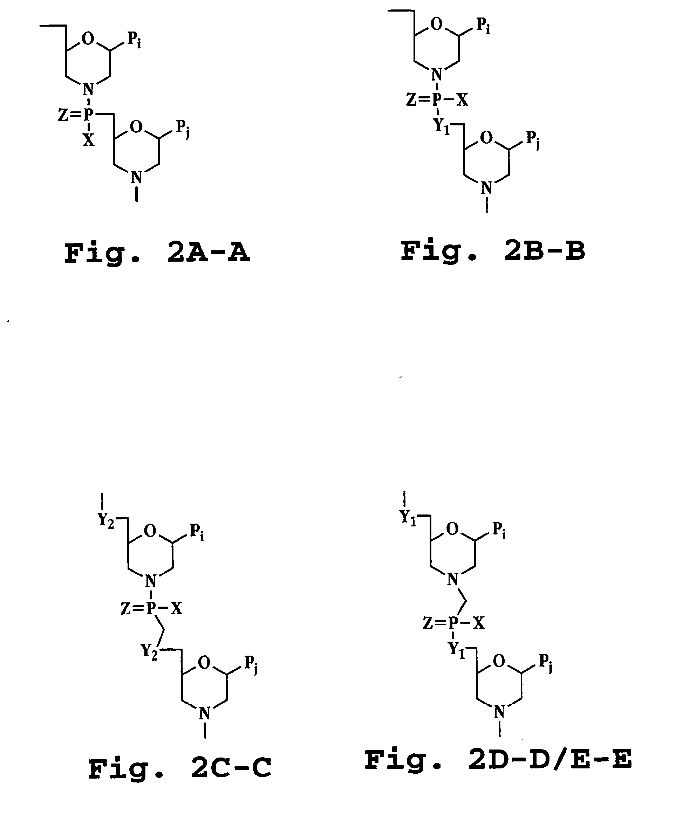

Subunit A in Figure 1 contains a 1-atom phosphorous-containing linkage which forms the five atom repeating-unit backbone shown at A-A in Figure 2, where the morpholino rings are linked by a 1-atom phosphoamide linkage.

Subunit B in Figure 1 is designed for 6-atom repeating-unit backbones, as shown at B-B, in Figure 2. In structure B, the atom Y linking the 5' morpholino carbon to the phosphorous group may be sulfur, nitrogen, carbon or, preferably, oxygen. The X moiety pendant from the phosphorous may be any of the following: fluorine; an alkyl or substituted alkyl; an alkoxy or substituted alkoxy; a thioalkoxy or substituted thioalkoxy; or, an unsubstituted, monosubstituted, or disubstituted nitrogen, including cyclic structures.

Subunits C-E in Figure 1 are designed for 7-atom unit-length backbones as shown for C-C through E-E in Figure 2. In Structure C, the X moiety is as in Structure B and the moiety Y may be a methylene, sulfur, or preferably oxygen. In Structure D the X and Y moieties are as in Structure B. In Structure E, X is as in Structure B and Y is O, S, or NR. In all subunits depicted in Figures 1A-E, Z is O or S, and Pᵢ or Pⱼ is adenine, cytosine, guanine or uracil.

A preferred "morpholino" oligonucleotide is composed of morpholino subunit structures of the form shown in Fig. 2B-B, where (i) the structures are linked together by phosphorodiamidate containing linkages, one to three atoms long, joining the morpholino nitrogen of one subunit to the 5' exocyclic carbon of an adjacent subunit, (ii) Pᵢ and Pⱼ are purine or pyrimidine base-pairing moieties effective to bind, by base-specific hydrogen bonding, to a base in a polynucleotide, and X = NH ₂, Y = O, and Z=O.

As noted above, the compound has a sequence which spans the start codon of a *c-myc* mRNA, meaning the compound contains a sequence complementary to a region of *c-myc* RNA containing the AUG mRNA start site and adjacent 5' and 3' base(s). The region of the mRNA against which the compound is directed is also referred to herein as the target sequence. The *c-myc* mRNA to which the antisense binds may be preprocessed (prespliced) mRNA, in which case the antisense compound may act to interfere with correct splicing, leading to truncated forms of the translated protein, or may bind to the processed mRNA, leading to inhibition of translation.

The compound is designed to hybridize to *c-myc* mRNA, under physiological conditions with a Tm substantially greater than 37°C, *e.g.*, at least 50°C and preferably 60°C-80°C. Although the compound is not necessarily 100% complementary to the target sequence, it is effective to stably and specifically bind to the target sequence such that expression of the target sequence, is modulated. The appropriate length of the oligomer to allow stable, effective binding combined with good specificity is about 8 to 40 nucleotide base units, and preferably about 12-25 base units. Mismatches, if present, are less destabilizing toward the end regions of the hybrid duplex than in the middle. Oligomer bases that allow degenerate base pairing with target bases are also contemplated, assuming base-pair specificity with the target is maintained. The compound preferably contains internal 3-base triplet complementary to the AUG site, and bases complementary to one or more bases 5' and 3' to the start site. One preferred compound sequence is the 20mer identified as SEQ ID NO:1 and having the base sequence: 5'-ACG TTG AGG GGC ATC GTC GC-3', where the CAT triplet in the sequences binds to the AUG start site, the 6 bases 3' to the CAT sequence extend in the upstream (5') direction on the target, and the 11 bases 5' to the CAT sequence extend downstream on the target. This compound has enhanced solubility by virtue of having no self-annealing regions. The solubility of the antisense compound, and the ability of the compound to resist precipitation on storage in solution, can be further enhanced by derivatizing the oligomer with a solubilizing moiety, such as a hydrophilic oligomer, or a charged moiety, such as a charged amino acid or organic acid. The moiety can be chemically attached to the antisense compound, *e*.*g*., at its 5' end, by well-known derivatization methods. One preferred moiety is a defined length oligo ethylene glycol moiety, such as triethyleneglycol, coupled covalently to the 5' end of the antisense compound through a carbonate linkage, via a piperazine linking group forming a carbamate linkage with triethyleneglycol, where the second piperazine nitrogen is coupled to the 5'-end phosphorodiamidate linkage of the antisense. Alternatively, or in addition, the compound may be designed to include one a small number of charged backbone linkages, such as a phosphodiester linkage, preferably near one of the ends of the compound. The added moiety is preferably effective to enhance solubility of the compound to at least about 30.mgs/ml, preferably at least 50 mgs/ml in aqueous medium.

The effectiveness of a particular *c-myc* antisense sequence may be determined by known screening methods. For example, the oligomer is incubated with a cell culture expressing the target RNA, and the presence or absence of the heteroduplex is determined by techniques such as those set forth in below, or by monitoring the presence or absence of the encoded, full-length protein as determined by standard techniques such as ELISA or Western blotting, or the presence or absence of active protein.

In another embodiment, the antisense compound forms part of a particle composition for use in restenosis treatment. One such particle is a biodegradable particle, *e*.*g*., a polylactate or polyglycolic particle, containing entrapped antisense compound. The particles are preferably in the 1-5 micron range, and are useful for delivery by direct particle delivery to an angioplasty vessel site, as described below, either by being impressed into the vessel walls by pressure from a balloon against the wall, or by release from a particle carrier, such as a stent.

Alternatively, the particles can be microbubbles containing the compound in entrapped form. The preparation of suitable microbubbles as antisense carrier is described, for example, by Porter et all cited above. The particles may be delivered directly to the vessel site, that is, by contacting the vessel walls with a directly with a suspension of the particles, with compound release from the particles when the vessel region is exposed to ultrasonic energy.

In still another embodiment the particles are liposomes containing entrapped antisense compound. Because the liposome particles are applied directly to the vessel site, the liposomes may be conventional liposomes without surface modifications needed for achieving long circulation times.

### III. Method of Treating Restenosis

Restenosis refers to the renarrowing of the vascular lumen following vascular intervention, such as coronary artery balloon angioplasty with or without stent insertion. It is clinically defined as greater than 50% loss of initial luminal diameter gain following the procedure. Restenosis is believed to occur in about 30% to 60% of lesions treated by angioplasty and about 20% of lesions treated with stents within 3 to 6 months following the procedure. (See, *e*.*g*., Dev).

"Restenosis" can also occur after a coronary artery bypass operation, wherein heart surgery is done to reroute, or "bypass," blood around clogged arteries and improve the supply of blood and oxygen to the heart. In such cases, the stenosis may occur in the transplanted blood vessel segments, and particularly at the junction of replaced vessels.

The present invention is directed to the use of a morpholino antisense compound for the preparation of a pharmaceutical composition for use in reducing the risk (incidence) or severity (extent of stenosis), particularly following balloon angioplasty, or in response to other vessel trauma, such as following an arterial bypass operation. The above-described antisense compound or composition is to be administered in an amount and via direct local administration of the compound at the vessel site of injury, to reduce the risk and/or severity of restenosis. In general, an amount of compound delivered to the vessel site between about 0.5-2 mg antisense compound is preferred, assuming substantially complete tissue uptake. Thus, where uptake into the vessel tissue is 10% of amount delivered, the amount delivered is preferably between 5 and 20 mg, preferably in a total volume of between about 0.2 to 1 ml.

In accordance with one aspect of the use, the modes of administration discussed below exploits one of more of the key features: (i) use of an antisense compound that has a high rate of cell uptake, (ii) the ability of the antisense compound to interfere with *c-myc* mRNA processing and mRNA translation, and (iii) local delivery of the compound by a mode of administration effective to achieve high localized concentration of the compound at the vessel injury site. The first two features have been discussed above. Modes of administration effective to achieve the third feature will now be detailed.

### A. Iontophoresis

In one embodiment of the invention the antisense compound is to be delivered by contacting the treated region with a reservoir containing an antisense compound and introducing the compound from the reservoir into the vessel by iontophoresis.

The antisense compounds described herein are uncharged. Optimal iontophoresis requires that the agent being administered have an overall net charge. The antisense compounds may be modified, as described above, to impart at least group that is charged at physiological or near-physiological pH. Alternatively, a pulsed electric field may be effective to facilitate the entry of uncharged antisense compounds into cells through an electroporesis effect.
Devices for use in carrying out iontophoretic drug delivery at a vessel site, *e*.*g*., by a balloon-catheter device have been described. In general, such devices include a reservoir for compound solution contained in a outer shell of the catheter's distal-tip balloon, an outer-balloon membrane allowing passage of the compound from the reservoir to the vessel wall, and an electrode communicating with the internal reservoir. A second counter-electrode is placed on the body, and a pulsed voltage is applied across the two electrodes to create a field that operates to draw charged compounds into vessel site. Devices, and electric pulse voltages and times follow those disclosed in the art, *e*.*g*., Fernandex-Ortiz, Dev, Robinson, and U.S. Pat Nos. 5,593,974, 5,628,730, and 5,425,703).

Alternatively, a pulsed-field device designed for diffusion or injection of uncharged compound into the site, with cell uptake facilitated by pulsed-field induced electroporation is also contemplated.

Both methods provide the advantages of high-efficiency delivery of antisense compound into the vessel-wall cells, without the need for high fluid pressure in introducing the compound into the vessel tissue. Assuming a desired dose of 1 mg for delivery to the vessel site, and an efficiency of tissue uptake of between 25-80 % , the total amount of compound contained in the reservoir for delivery is between about 1.25 and 4 mg, preferably at a concentration of between about 25-50 mgs/ml.

### B. Nipple Balloon Catheter or Infiltrator

In a second general compound-delivery approach, the compound is to be injected into the vessel, that it, below the vessel surface, by means of an injection balloon catheter, such has been described (*e*.*g*., Roy, Pavlides, and Barath). The catheter, which is known commercially as an "Infiltrator Angioplasty Balloon Catheter" or "IABC", is a balloon catheter with 3 lumens: one for inflating the balloon, one central for a guidewire, and a third for drug delivery. On the surface of the balloon there are several longitudinal strips or channels, each having a plurality of injection needles, *e*.*g*., six needles, which upon inflation stand project above the channel surface and are connected to the drug-delivery lumen. When the balloon inflates, the needles penetrate the lesion, allowing drug delivery into the tunica media of the vessel wall.

In the present invention, the reservoir is filled with an antisense composition preferably containing a compound concentration of about 25-50 mgs/ml. Assuming an uptake into tissue of between about 15-50 percent, the amount of material injected is in the range of about 0.04 ml to 0.25 ml. The relatively small volume of compound that is administered reduced the risk of further injury by fluid injection under pressure into the injury site.

This mode of administration provides the advantage of high efficiency of uptake of the compound into the vessel tissue (20% or greater).

### C. Hydrogel Coating

In a third delivery approach, the compound is embedded or dissolved in a diffusable medium, typically hydrogel, that coats the outer surface of a balloon, *e*.*g*., on a balloon catheter used for angioplasty. Methods for making and using such hydrogel coating on a catheter balloon have been described (*e*.*g*., Imanishi, Dick).

The hydrogel coating is formulated to include the antisense compound, at a preferred concentration of about 25-50 mgs/ml, and to release the selected dose of the compound for a period of about 5-60 minutes. The total amount of hydrogel is preferably between about 0.1 to 0.5 ml, allowing a total delivery of about 2.5 to 25 mgs, to accommodate an efficiency of tissue uptake of about 5-40%.
The hydrogel diffusion method may be combined with iontophoresis or electroporation, as described above, to enhance uptake of the compound from the gel into the tissue. In this case, the amount of material in the gel may be reduced substantially, in view of the enhanced efficiency of uptake.

The method has the advantages of maintaining intimate contact between the compound reservoir and vessel wall during the compound delivery period, allowing a relatively slow rate of drug release and uptake by cells, and avoiding elevated injection pressures.

### D. Stents

This approach is similar to the hydrogel method above, except that the compound is contained in diffusable form in a coating contained on an intravascular stent. The stent may be placed at the vessel site at the time of balloon angioplasty, or placed at the site during coronary bypass surgery. Stent designs and materials, including biodegradable stents which release compound upon biodegradation, or which include a coating containing the compound in diffusable form, are known (Raman and U.S. Pat Nos: 5,997,468 and 5,871,535).

As above, the stent or stent coating contains an amount of drug sufficient to deliver an approximately 0.5-2 mg dose over a 5-60 minute period, with an expected efficiency of uptake into tissue between 5-20 percent.

An implanted stent provides two advantages in practicing the present invention. First, it allows short term dosing, as with the other methods, and also continued dosing at a lower level over an extended period, *e*.*g*., 1-14 days, to block the early events of restenosis. Secondly, the stent itself may be effective in reducing the risk of restenosis, as has been reported.

### E. Microparticles.

Microparticles, such as polystyrene microparticles (Seradyn, Indianapolis, Ind.), biodegradable particles, liposomes or microbubbles containing the antisense compound in releasable form may be used for direct delivery of the compound into the vessel tissue.

The particles are prepared to contain a total dose of preferably 0.5-2 mg, with the total does depending on the efficiency of tissue uptake. Where the particles are injected into the tissue, this uptake will be high, *e*.*g*., 30-70% or higher. Where the particles are merely brought into contact with the vessel wall, the uptake of compound will be lower.

Methods for delivery the particles include injection of a particle suspension, or physical pressing the particles against the vessel wall, *e*.*g*., by balloon pressure in a balloon containing a outer coating of particles, *e*.*g*., in a hydrogel medium, or by embedding the particles in releasable form in a stent. Where the particles are microbubbles, the method additional includes exposing the administered particles to ultrasonic energy to explode the bubbles and release the bubbles at the particle sites. Particle delivery of the compound has the advantage of high uptake, particular where the particles are injected, and the potential for both high, short-term drug release and extended release from depot-release particles, *e*.*g*., biodegradable particles. The particles may also be coated with a binding agent, *e*.*g*., antibodies specific against growth factors or other proteins that are actively synthesized by endothelial cells during early cellular events leading to restenosis (see Bauriedel), to enhance the efficiency of compound uptake. Finally, the antisense compound may be selectively released from the particles at a desired time, as in the case for microbubbles.

### IV. Restenosis Method

In a related aspect, the invention includes the use of a morpholino antisense compound for the preparation of a pharmaceutical composition for treating the risk of restenosis in a region of a patient's coronary vessel. The method is to be carried out by administering to the patient, by local delivery directly into the region of injury, a morpholino antisense compound having (i) the base sequence identified as SEQ ID NO:1, (ii) a phosphorodiamidate backbone shown in. Figure 2B-B, where X=NH ₂, Y=O, and Z=O, and (iii) a moiety that enhances the solubility of the compound, preferably to a solubility in aqueous medium of between 25-50 mgs/ml or greater. The administration is by direct contact with the vessel, using methods described above, or alternative methods, such as direct injection of the material through a Wilinsky type balloon catheter having a drug-solution reservoir, and means for injecting the solution through pores in the balloon against the vessel walls. The amount of material injected is preferably designed to provide a dose of material taken up by the tissue of between 0.5 to 2 mg antisense compound.

The moiety that increases compound solubility is a biocompatible hydrophilic or charged moiety that can be coupled to the antisense compound, and which does not interfere with compound binding to the target sequence, i.e. a triethyleneglycol moiety derivatized to the antisense compound through a carbamate-piperizine linkage as described above.

### V. Method of Assaying effectiveness of antisense delivery and uptake.

A standard indicator of the success of PTCA is one or more follow-up angiograms to determine the minimal lumen diameter of the affected vessel, that is, the extent of reocclusion. In determining the success of the methods of the present invention, follow-up angiograms may be completed one or more times following implantation of the *c-myc* antisense-containing catheter. Indicators of successful therapeutic intervention include a low percent occurrence of re-occlusion and/or restenosis and a prolonged time to occurrence of re-occlusion and/or restenosis.

Further described is a rapid, easily performed method for confirming the presence of *c-myc* antisense compound in target cells, following antisense administration at the vessel site, and for comparing uptake levels of the compound achieved by various methods of compound administration to optimize conditions and dosages for effective restenosis treatment.

The method is based on the discovery, disclosed in above-cited U.S. provisional application 60/117,846 for "Non-Invasive Method for Detecting RNA", that a morpholino antisense compound of the type disclosed herein, when administered *in vivo*, can be detected in the urine of the receiving subject in a heteroduplex form consisting of the antisense compound and its RNA complement. The data indicate a sequence of events that include (i) uptake of the antisense compound by cells in the subject, (ii) binding of the compound intracellularly with the target mRNA, (iii) intracellular nuclease cleavage of single-stranded portions of the antisense/target complex, leaving a heteroduplex consisting of the antisense and its mRNA complement; (iv) secretion of the heteroduplex, presumably recognized as foreign molecules, by the cells, and (v) appearance of the heteroduplex in the blood and eventually the urine.

In the present case, this sequence of events allows for one to administer *c-myc* antisense, in accordance with any of the methods detailed above, and follow the uptake of the compound into target cells, by monitoring the presence and or quantity of *c-myc* antisense/mRNA in the urine or other body fluid, *e*.*g*., blood or serum.

In practicing the method, the antisense compound of the invention is administered to a patient or in an animal model in a selected dose, and by a selected mode of delivery, including any of the ones mentioned above. Thereafter, and at selected times after administration, *e*.*g*., 4, 12, and 24 hours post administration, the urine is monitored for the appearance and/or amount of heteroduplex to determine the effectiveness of compound uptake at the selected dose and method of administration.

In one exemplary assay format for use in urine detection, a sample containing an antisense/:RNA heteroduplex is reacted with a compound that specifically binds to or modifies the oligomer:RNA heteroduplex (*e*.*g*., a monoclonal antibody (mAb) specific for the particular heteroduplex) followed by detection of the modified or conjugated oligomer:RNA heteroduplex.

In another exemplary assay format, an antisense oligomer is modified by conjugating it with a reporter molecule before administration to the subject, followed by separation of heteroduplexes from uncomplexed reporter labeled antisense oligomer and detection of the heteroduplex-associated reporter molecule. In some cases such separation may be carried out by via chromatography or electrophoresis.

Exemplary detection methods include spectrophotometric detection (*e*.*g*., with a fluorescence detector), or detection using antibodies (*e*.*g*., FACS analysis). Such methods may be combined with separation methods in order to expedite analysis, *e*.*g*. chromatographic separation with simultaneous fluorescence detection or electrophoretic separation with detection by staining of gels, fluorescence or autoradiographic detection. Such techniques are known to those of skill in the art and readily adaptable to a given antisense oligomer and target RNA sequence.

Any fluorescent molecule known in the art for labeling nucleic acids may be used in the described methods, for example, fluorescein and fluorescein derivatives such as carboxy fluorescein, 5-(4,6-dichlorotriazin-2-yl) amino fluorescein (5-DTAF); eosin; rhodamines such as Texas Red and tetramethylrhodamine: cyanine dyes such as thiazole orange, oxazole yellow and related dyes described in U.S. Pat. Nos. 4,957,870 and 4,888,867; pyrene; porphyrin dyes such as La JollaBlue. The fluorescent label should be selected such that its fluorescent lifetime is comparable in magnitude to the correlation time being measured, taking into account that temperature, viscosity, and the size of the oligonucleotide to which the fluorescent dye is conjugated all affect tumbling time. The fluorescent label is covalently linked or conjugated to the signal primer so as not to interfere with either emission of fluorescence from the label or hybridization of the probe to the target sequence. [See, also, U.S. Pat. No. 5,614,617 and 5,652,099.]

In other cases, antisense oligomers can be synthesized having a sequence complementary to a given target with the 5' end of the sequence attached to a reactive amino group as described by Smith, L. M., et al. Nuc. Acids Res. 13(7):2399 (1985). In such cases, biotin, peptide or an enzyme, *e*.*g*., alkaline phosphatase may be attached to the 5' amino group. [See, also U.S. Pat. No. 5,783,391.)

In still another embodiment, the heteroduplex can be detected, *e*.*g*., after isolation from the body-fluid sample, by mass spectroscopy. In studies conducted in support of the present invention, it was found that a heteroduplex of RNA:morpholino oligomer is readily resolved into two different-MW fractions (the two heteroduplex strands) by mass spectroscopy. This method thus provides a positive identification of the heteroduplex in terms of its two component strands.

As can be appreciated from above, the method allows one to readily assess the effectiveness of various modes, of administration, and optimal doses, typically doses that lead to maximal or near-maximal levels of heteroduplex in the urine. This will allow a physician to monitor the effectiveness of the treatment method and assure the physician that the antisense compound has been taken up by the vessel tissue. If, for example, the test shows low levels of heteroduplex after 24 hours, the physician might deem it necessary to retreat the site.

The following example illustrates the basic features of the assay method.

### Example 1

### In vivo studies with antisense oligomer:RNA heteroduplexes

Calibration studies performed using an instrument capable of detecting fluorescein conjugated oligomers (Applied Biosystems Model 672 GeneScanner) were used to determine the migration rates of fluorescein-conjugated oligomers of various lengths; a 15-mer, a 20-mer, a 24-mer and a 38-mer ribozyme. Concentrations were evaluated in a GeneScanner.

Rats were injected with carboxyfluorescein-conjugated phosphorodiamidate morpholino oligomers (PMO) which is antisense to rat cytochrome P-4503A2.

Chromatograms of plasma samples prepared from blood withdrawn at the various times post- PMO administration showed the following. Plasma samples prepared from rats one hour post-injection contained fluorescent components which migrated at 270 and 340 minutes (two peaks due to the two possible carboxyfluorescein linkages which migrate differently). Plasma samples prepared from rats 24 hours post-injection contained fluorescent components which migrated at approximately 75 and 80 minutes. Mass spectral data (not shown) confirms that the shorter migration time is not due to degradation of the PMO and indicates that a PMO:RNA heteroduplex has been formed over that time.

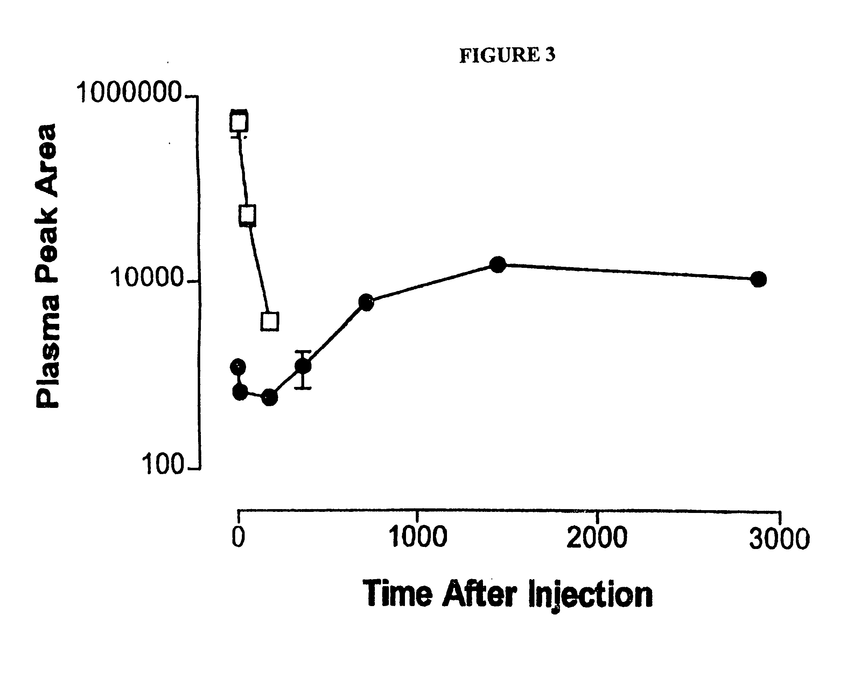

Figure 3 represents samples taken at various times (in minutes) post administration of the P450 antisense PMO, and indicates the disappearance of the PMO monomer (open squares) and the corresponding appearance of RNA:PMO heterodimer (solid circles) in the plasma of rats following such administration. Appearance of significant quantities of the duplex in plasma does not occur until the majority of the unduplexed PMO leaves the plasma in what is generally referred to as the "distribution phase". The PMO heteroduplex does not accumulate in plasma until after PMO monomer has distributed into the tissues of the subject where the complementary mRNA transcripts are localized. The charged RNA:PMO duplex presumably forms in these tissues and effluxes out of cells and back into plasma. This overall process requires several hours.

After administration of the p450 antisense PMO, fluorescein was detected in both the kidney and liver. Chromatograms of kidney tissue sample shows a band at 350 minutes consistent with unduplexed PMO and an additional band at 80 minutes consistent with the PMO:RNA heteroduplex, indicating both duplex and parent PMO which may reside in interstitial spaces or within the cells of the kidney. The liver tissue sample shows essentially no unduplexed PMO and significantly more PMO:RNA heteroduplex. These results are consistent with the observation that levels of P450 mRNA transcript are much lower in kidney than in liver.

Studies reflecting the time course of urinary clearance of unduplexed antisense PMO oligomer and antisense PMO oligomer:RNA heteroduplexes indicate that several hours are required for formation and efflux of PMO:RNA heteroduplex from tissues into plasma, followed by their ultimate appearance in urine.

## Claims

1. Use of a morpholino antisense compound having:
(i) from 8 to 40 nucleotides,
(ii) morpholino subunits linked together by phosphorodiamidate linkages, 2 atoms long, joining the morpholino nitrogen of one subunit to the 5' exocyclic carbon of an adjacent subunit, and
(iii) a sequence of bases attached to the subunits and containing the sequence identified by SEQ ID NO:1,
for the preparation of a pharmaceutical composition for use in inhibiting restenosis in a patient.

2. The use of claim 1 wherein the morpholino antisense compound has intersubunit linkages of the form: 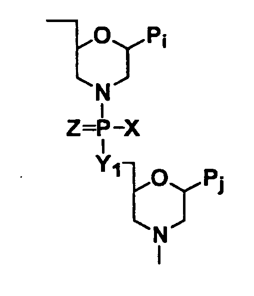 where X is an unsubstituted, monosubstituted or disubstituted nitrogen, Y₁ and Z are oxygen and Pᵢ and Pⱼ are purine or pyrimidine base-pairing moieties effective to bind, by base-specific hydrogen bonding, to a base in a polynucleotide.

3. The use of claim 1, wherein the antisense compound has from 8 to 40 nucleotides, including a targeting sequence complementary to a region spanning the start codon of human *c-myc* mRNA, where the nucleotide sequence is SEQ ID NO:1, for the preparation of a pharmaceutical composition to be administered directly to a vessel site.

4. The use of claim 3, wherein the pharmaceutical composition is to be administered by:
(a) contacting the region of the vessel with a reservoir containing the antisense compound, and introducing the compound from the reservoir into the vessel by iontophoresis or electroporation;
(b) injecting the compound from the catheter directly into the region of the vessel, under pressure, through injectors contained on the surface of the catheter balloon, where said injectors are capable of penetrating the tunica media in the vessel;
(c) injecting into or contacting the region of the vessel, microparticles containing the antisense compound in entrapped form;
(d) contacting the region of the vessel with a hydrogel coating contained on the surface of the catheter balloon, and containing the antisense compound in diffusible form; or
(e) contacting the region of the vessel with a stent having an outer surface layer containing the antisense compound in diffusible form.

5. The use of claim 4, wherein the amount of antisense compound to be administered is between 0.5 and 20 mg.

6. The use of claim 4, wherein in alternative (a) the antisense compound is contained in a volume between two inflated balloons in the catheter, the compound contains a net charge, and the volume is subjected to pulsed electric fields effective to iontophoretically drive the compound into region of the vessel.

7. The use of claim 4, wherein in alternative (a) the antisense compound is contained in a volume between two inflated balloons in the catheter, and the volume is subjected to pulsed electric fields effective to facilitate compound uptake into vessel-region cells by electroporation.

8. The use of claim 4, wherein in alternative (b) the catheter balloon has a plurality of outer-facing channels that communicate with a distal-tip reservoir, each channel having one or more injection ports, and said injecting includes forcing a solution or suspension of the antisense compound from said reservoir through said injection ports when the balloon is in an inflated position.

9. The use of claim 4, wherein in alternative (c) the catheter has a distal end reservoir, the microparticles are contained as a particle suspension in the reservoir, and said injecting includes forcing the suspension out of the catheter through a catheter surface in contact with the vessel region.

10. The use of claim 4, wherein the particles are microbubbles containing the antisense compound in entrapped form.

11. The use of claim 4, wherein in alternative (e) the stent is biodegradable.

12. The use of claim 4, wherein the compound is derivatized with triethyleneglycol attached to the 5' end of the compound, and the compound is to be administered from a solution containing at least 30 mg/ml of the antisense compound.

13. A morpholino antisense compound having:
(i) from 8 to 40 nucleotides,
(ii) morpholino subunits linked together by phosphorodiamidate linkages, 2 atoms long, joining the morpholino nitrogen of one subunit to the 5' exocyclic carbon of an adjacent subunit, and
(iii) a sequence of bases attached to the subunits and containing the sequence identified by SEQ ID NO:1.

14. A morpholino antisense compound having (i) from 8 to 40 nucleotides, including a targeting nucleic acid sequence complementary to a region that spans the start codon of a human *c-myc* mRNA gene, and (ii) morpholino subunits linked together by phosphorodiamidate linkages, 2 atoms long, joining the morpholino nitrogen of one subunit to the 5' exocyclic carbon of an adjacent subunit.

15. The compound of claim 13 having intersubunit linkages of the form: 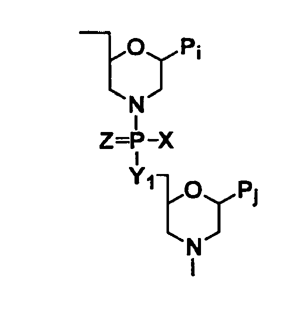 where X is an unsubstituted, monosubstituted or disubstituted nitrogen, Y₁ and Z are oxygen and Pᵢ and Pⱼ are purine or pyrimidine base-pairing moieties effective to bind, by space-specific hydrogen bonding, to a base in a polynucleotide.

16. The compound of claim 13, wherein the compound is derivatized with triethyleneglycol attached to the 5' end of the compound.

17. The compound of claim 13, which is entrapped in liposomal or biodegradable microparticles.

## Patentansprüche

1. Verwendung einer Morpholino-Antisenseverbindung mit
(i) 8 bis 40 Nucleotiden,
(ii) Morpholino-Untereinheiten, die untereinander durch Phosphorodiamidat-Verknüpfungen verbunden sind, die 2 Atome lang sind und den Morpholino-Stickstoff einer Untereinheit mit dem 5'-exocyclischen Kohlenstoff einer benachbarten Untereinheit verbinden, und
(iii) einer Basensequenz, die an die Untereinheiten angehängt ist und die die Sequenz enthält, die durch SEQ ID NO: 1 identifiziert ist,
für die Herstellung eines Arzneimittels zur Verwendung in der Hemmung von Restenose in einem Patienten.

2. Verwendung gemäß Anspruch 1, wobei die Morpholino-Antisenseverbindung Verknüpfungen zwischen den Untereinheiten der Form: 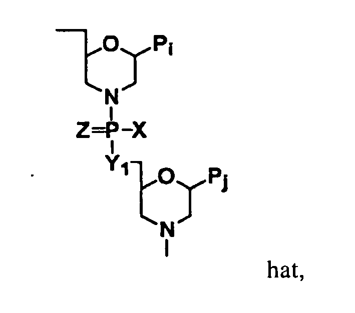 wobei X ein unsubstituierter, monosubstituierter oder disubstituierter Stickstoff ist, Y₁ und Z Sauerstoff sind und Pᵢ und Pⱼ Purin- oder Pyrimidinbasenpaar-bildende Einheiten sind, die wirksam sind, durch basenspezifische Wasserstoffbindung an eine Base in einem Polynucleotid zu binden.

3. Verwendung gemäß Anspruch 1, wobei die Antisenseverbindung 8 bis 40 Nucleotide hat, einschließlich einer Targetingsequenz, die komplementär zu einem Bereich ist, der sich über das Startcodon einer menschlichen c-myc-mRNA erstreckt, wobei die Nucleotidsequenz SEQ ID NO:1 ist, für die Herstellung eines Arzneimittels, das direkt an eine Blutgefäßstelle verabreicht werden soll.

4. Verwendung gemäß Anspruch 3, wobei das Arzneimittel verabreicht werden soll durch:
(a) Inkontaktbringen des Blutgefäßbereiches mit einem Reservoir, das die Antisenseverbindung enthält, und Einbringen der Verbindung vom Reservoir in das Blutgefäß durch Iontophorese oder Elektroporation;
(b) Injizieren der Verbindung unter Druck vom Katheter direkt in den Blutgefäßbereich durch Injektoren, die auf der Oberfläche des Katheterballons enthalten sind, wobei die Injektoren fähig sind, die Tunica media in dem Blutgefäß zu durchdringen;
(c) Injizieren oder Inkontaktbringen des Blutgefäßbereichs mit Mikropartikeln, die die Antisenseverbindung in eingeschlossener Form enthalten;
(d) Inkontaktbringen des Blutgefäßbereichs mit einer Hydrogelbeschichtung, die auf der Oberfläche des Katheterballons enthalten ist, und die die Antisenseverbindung in diffusionsfähiger Form enthält; oder
(e) Inkontaktbringen des Blutgefäßbereichs mit einem Stent, der eine äußere Oberflächenschicht hat, die die Antisenseverbindung in diffusionsfähiger Form enthält.

5. Verwendung gemäß Anspruch 4, wobei die Menge der Antisenseverbindung, die verabreicht werden soll, zwischen 0,5 und 20 mg liegt.

6. Verwendung gemäß Anspruch 4, wobei bei Variante (a) die Antisenseverbindung in einem Volumen zwischen zwei aufgeblasenen Ballons in dem Katheter enthalten ist, die Verbindung eine Nettoladung enthält und das Volumen gepulsten elektrischen Feldern ausgesetzt ist, die wirksam sind, die Verbindung iontophoretisch in den Blutgefäßbereich zu lenken.

7. Verwendung gemäß Anspruch 4, wobei in Variante (a) die Antisenseverbindung in einem Volumen zwischen zwei aufgeblasenen Ballons in dem Katheter enthalten ist und das Volumen gepulsten elektrischen Feldern ausgesetzt ist, die wirksam sind, die Aufnahme der Verbindung in Zellen des Blutgefäßbereichs durch Elektroporation zu erleichtern.

8. Verwendung gemäß Anspruch 4, wobei in Variante (b) der Katheterballon eine Vielzahl von äußeren Kanälen hat, die mit einem Reservoir am distalen Ende in Verbindung stehen, wobei jeder Kanal eine oder mehrere Injektionsöffnungen hat und das Injizieren das Vorantreiben einer Lösung oder Suspension der Antisenseverbindung vom Reservoir durch die Injektionsöffnungen einschließt, wenn der Katheter in einer aufgeblasenen Position ist.

9. Verwendung gemäß Anspruch 4, wobei in Variante (c) der Katheter ein Reservoir am distalen Ende hat, die Mikropartikel als eine Partikelsuspension in dem Reservoir enthalten sind und das Injizieren das Austreiben der Suspension aus dem Katheter durch eine Katheteroberfläche, die mit dem Blutgefäßbereich in Kontakt ist, einschließt.

10. Verwendung gemäß Anspruch 4, wobei die Partikel Mikroblasen sind, die die Antisenseverbindung in eingeschlossener Form enthalten.

11. Verwendung gemäß Anspruch 4, wobei in Variante (e) der Stent biologisch abbaubar ist.

12. Verwendung gemäß Anspuch 4, wobei die Verbindung mit Triethylenglykol derivatisiert ist, das an das 5'-Ende der Verbindung angehängt ist, und die Verbindung von einer Lösung verabreicht werden soll, die mindestens 30 mg/ml der Antisenseverbindung enthält.

13. Morpholino-Antisenseverbindung mit
(i) 8 bis 40 Nucleotiden,
(ii) Morpholino-Untereinheiten, die untereinander durch Phosphorodiamidat-Verknüpfungen verbunden sind, die 2 Atome lang sind und den Morpholino-Stickstoff einer Untereinheit mit dem 5'-exocyclischen Kohlenstoff einer benachbarten Untereinheit verbinden und
(iii) einer Basensequenz, die an die Untereinheiten angehängt ist und die die Sequenz enthält, die durch SEQ ID NO: 1 identifiziert ist.

14. Morpholino-Antisenseverbindung mit (i) 8 bis 40 Nucleotiden, einschließlich einer Targeting-Nucleinsäuresequenz, die komplementär zu einem Bereich ist, der sich über das Startcodon eines menschlichen c-myc-mRNA-Gens erstreckt, und (ii) Morpholino-Untereinheiten, die untereinander durch Phosphorodiamidat-Verknüpfungen verbunden sind, die 2 Atome lang sind und den Morpholino-Stickstoff einer Untereinheit mit dem 5'-exocyclischen Kohlenstoff einer benachbarten Untereinheit verbinden.

15. Verbindung gemäß Anspruch 13 mit Verknüpfungen zwischen den Untereinheiten der Form: 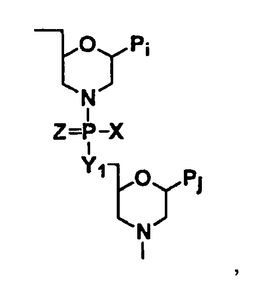 wobei X ein unsubstituierter, monosubstituierter oder disubstituierter Stickstoff ist, Y₁ und Z Sauerstoff sind und Pᵢ und Pⱼ Purin- oder Pyrimidinbasenpaar-bildende Einheiten sind, die wirksam sind, durch basenspezifische Wasserstoffbindung an eine Base in einem Polynucleotid zu binden.

16. Verbindung gemäß Anspruch 13, wobei die Verbindung mit Triethylenglykol, das an das 5'-Ende der Verbindung angehängt ist, derivatisiert ist.

17. Verbindung gemäß Anspruch 13, die in liposomalen oder biologisch abbaubaren Mikropartikeln eingeschlossen ist.

## Revendications

1. Utilisation d'un composé morpholino antisens ayant :
(i) de 8 à 40 nucléotides,
(ii) des sous-unités morpholino reliées entre elles par des liaisons phosphorodiamidate, d'une longueur de 2 atomes, liant l'azote morpholino d'une sous-unité au carbone 5'-exocyclique d'une sous-unité adjacente, et
(iii) une séquence de bases fixée aux sous-unités et contenant la séquence identifiée par la SEQ ID N°1,
pour la préparation d'une composition pharmaceutique destinée à être utilisée dans l'inhibition de la resténose chez un patient.

2. Utilisation selon la revendication 1, dans laquelle le composé morpholino antisens a des liaisons entre sous-unités de la forme : 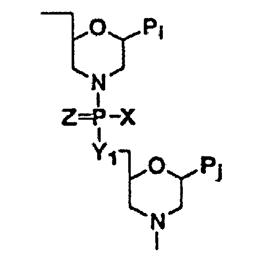 dans laquelle X est un atome d'azote non substitué, monosubstitué ou disubstitué, Y₁ et Z sont des atomes d'oxygène et Pᵢ et Pⱼ sont des entités s'appariant efficacement aux bases purines ou pyrimidines pour se lier, par liaison hydrogène base spécifique, à une base dans un polynucléotide.

3. Utilisation selon la revendication 1, dans laquelle le composé antisens a de 8 à 40 nucléotides, y compris une séquence de ciblage complémentaire à une région englobant le codon de départ de l'ARNm humain *c-myc*, où la séquence de nucléotides est la SEQ ID N°1, pour la préparation d'une composition pharmaceutique destinée à être administrée directement sur un site vasculaire.

4. Utilisation selon la revendication 3, dans laquelle la composition pharmaceutique est destinée à être administrée :
(a) en mettant en contact la région du vaisseau avec un réservoir contenant le composé antisens et en introduisant le composé du réservoir dans le vaisseau par iontophorèse ou électroporation ;
(b) en injectant le composé depuis le cathéter directement dans la région du vaisseau, sous pression, par le biais d'injecteurs présents à la surface du ballonnet du cathéter, où lesdits injecteurs sont capables de pénétrer dans la média du vaisseau ;
(c) en injectant dans la région du vaisseau ou en mettant en contact avec celle-ci des microparticules contenant le composé antisens sous forme piégée ;
(d) en mettant en contact la région du vaisseau avec un revêtement hydrogel présent à la surface du ballonnet du cathéter, et contenant le composé antisens sous forme diffusable ; ou
(e) en mettant en contact la région du vaisseau avec une endoprothèse vasculaire ayant une couche superficielle extérieure contenant le composé antisens sous forme diffusable.

5. Utilisation selon la revendication 4, dans laquelle la quantité de composé antisens à administrer est située entre 0,5 et 20 mg.

6. Utilisation selon la revendication 4, dans laquelle, en variante de (a), le composé antisens est contenu dans un volume entre deux ballonnets gonflés dans le cathéter, le composé contient une charge nette, et le volume est soumis à des champs électriques pulsés efficaces pour conduire le composé dans la région du vaisseau par iontophorèse.

7. Utilisation selon la revendication 4, dans laquelle, en variante de (a), le composé antisens est contenu dans un volume entre deux ballonnets gonflés dans le cathéter, et le volume est soumis à des champs électriques pulsés efficaces pour faciliter l'absorption du composé dans les cellules de la région vasculaire par électroporation.

8. Utilisation selon la revendication 4, dans laquelle, en variante de (b), le ballonnet du cathéter a une pluralité de canaux faisant face vers l'extérieur, qui communiquent avec un réservoir à embout distal, chaque canal ayant un ou plusieurs orifices d'injection, et ladite injection comprend le forçage d'une solution ou d'une suspension du composé antisens à partir dudit réservoir par le biais desdits orifices d'injection quand le ballonnet est dans une position gonflée.

9. Utilisation selon la revendication 4, dans laquelle, en variante de (c), le cathéter a un réservoir terminal distal, les microparticules sont contenues sous forme d'une suspension de particules dans le réservoir, et ladite injection comprend le forçage de la suspension hors du cathéter par le biais d'une surface de cathéter en contact avec la région du vaisseau.

10. Utilisation selon la revendication 4, dans laquelle les particules sont des microbulles contenant le composé antisens sous forme piégée.

11. Utilisation selon la revendication 4, dans laquelle, en variante de (e), l'endoprothèse vasculaire est biodégradable.

12. Utilisation selon la revendication 4, dans laquelle le composé est dérivatisé avec un triéthylène glycol fixé à l'extrémité 5' du composé et le composé est destiné à être administré à partir d'une solution contenant au moins 30 mg/ml de composé antisens.

13. Composé morpholino antisens ayant :
(i) de 8 à 40 nucléotides,
(ii) des sous-unités morpholino reliées entre elles par des liaisons phosphorodiamidate, d'une longueur de 2 atomes, liant l'azote morpholino d'une sous-unité au carbone 5'-exocyclique d'une sous-unité adjacente, et
(iii) une séquence de bases fixée aux sous-unités et contenant la séquence identifiée par la SEQ ID N°1.

14. Composé morpholino antisens ayant (i) de 8 à 40 nucléotides, comprenant une séquence d'acide nucléique de ciblage complémentaire avec une région qui englobe le codon de départ d'un gène d'ARNm humain *c-myc*, et (ii) des sous-unités morpholino reliées entre elles par des liaisons phosphorodiamidate, d'une longueur de 2 atomes, liant l'azote morpholino d'une sous-unité au carbone 5'-exocyclique d'une sous-unité adjacente.

15. Composé selon la revendication 13, ayant des liaisons entre sous-unités de la forme : 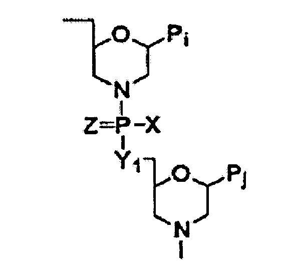 dans laquelle X est un atome d'azote non substitué, monosubstitué ou disubstitué, Y₁ et Z sont des atomes d'oxygène et Pᵢ et Pⱼ sont des entités s'appariant efficacement aux bases purines ou pyrimidines pour se lier, par liaison hydrogène base spécifique, à une base dans un polynucléotide.

16. Composé selon la revendication 13, dans lequel le composé est dérivatisé avec le triéthylène glycol fixé à l'extrémité 5' du composé.

17. Composé selon la revendication 13, qui est piégé dans des microparticules liposomales ou biodégradables.
